# EUROPEAN PATENT APPLICATION

(11) **EP 3 698 756 A1**
(43) Date of publication of application: **26.08.2020**
(21) Application number: 18868159.7
(22) Date of filing: 16.10.2018
(51) Int. Cl.: A61F 2/07, A61M 29/00

(54) **STENT GRAFT**

(30) Priority: 16.10.2017 JP 2017200237
(71) Applicant: Kawasumi Laboratories, Inc., Saiki-shi, Oita 876-0121 (JP)
(72) Inventor: YOKOTA Tomoaki, Bungo-ono-shi Oita 879-7153 (JP)
(74) Representative: LKGLOBAL Lorenz & Kopf PartG mbB Patentanwälte
(86) International application number: PCT/JP2018/038547
(87) International publication number: WO 2019/078218

(57) **Abstract**

Provided is a stent graft (1) comprising: a framework portion (B) having a ductal shape and which is expandable and contractable radially of the stent graft, and a membrane portion (GF) disposed along the framework portion. The membrane portion (GF) comprises a bent guide portion (RU) comprising a regular arrangement of textured structures formed radially of the stent graft. The bent guide portion is composed so as to have priority over other sections of the membrane portion in deforming when the stent graft bends.

## Description

### TECHNICAL FIELD

The present invention relates to a stent graft.

### BACKGROUND ART

Conventionally, there have been known stent grafts which are placed in living body lumens such as blood vessels. A stent graft generally has a ductal shape, and includes a radially expandable and contractable framework portion (i.e. stent), and a membrane portion (i.e. graft) formed along the framework portion.

By modularization of a framework portion, one of the conventional stent grafts improves followability to living body lumens which have various shapes and may cause deformation of pulsation or the like in association with biological activity. Specifically, plural types of preparatory framework bodies having different radial thicknesses, axial lengths, and the like are previously prepared, the plurality of the preparatory framework bodies are selected and assembled in accordance with a shape of a living body lumen where the stent graft is placed, so that a framework portion adapted to the shape of the living body lumen is configured (e.g. see Patent Document 1).

### PRIOR ART DOCUMENT

### PATENT DOCUMENT

Patent Document 1: Japanese Patent Publication No. 4064724

### SUMMARY OF THE INVENTION

### [Technical Problem]

Although the aforementioned conventional stent graft is excellent in followability to living body lumens, it is not suitable for mass production because each framework portion is individually manufactured for each actual operation, and therefore it is difficult to increase a productivity. On the other hand, if stent grafts having the same structure are mass-produced, the productivity is excellent, but the stent grafts may not sufficiently follow a living body lumen depending on a shape or deformation of the living body lumen where the stent graft is placed. Excessive shortage in the followability to the living body lumen may cause collapse (so-called kink) of the framework portion, separation of the stent graft from an inner wall of the living body lumen, or the like.

One of the objects of the present invention is to provide a stent graft which can achieve both followability to a living body lumen and productivity in manufacture.

### SOLUTION TO PROBLEM

In one aspect of the present invention, the stent graft has a ductal shape, the stent graft including a framework portion expandable and contractable along a radial direction of the stent graft, and a membrane portion provided along the framework portion, in which the membrane portion includes a bent guide portion which has a textured structure formed in the radial direction being regularly arranged and is deformable more preferentially than other positions in the membrane portion when the stent graft bends.

### ADVANTAGEOUS EFFECT OF THE INVENTION

According to the present invention, followability to a living body lumen can be improved by the bent guide portion of the membrane portion. In addition, unlike the conventional stent graft, each framework portion does not need to be individually manufactured for each living body lumen where the stent graft is placed, and productivity of the stent graft can be improved. That means, both followability to the living body lumen and productivity in manufacture can be achieved.

### BRIEF DESCRIPTION OF THE DRAWINGS

FIG. 1 is a schematic drawing of a stent graft according to an embodiment of the present invention.
FIG. 2 (A) is an enlarged drawing of the stent graft illustrated in FIG. 1 and FIG. 2 (B) to FIG. 2 (D) are sectional drawings of the stent graft illustrated in FIG. 2 (A).
FIG. 3 (A) and FIG. 3 (B) are enlarged drawings of a framework portion (specifically, strut) of the stent graft illustrated in FIG. 2.
FIG. 4 is a developed drawing of a membrane portion used for the stent graft illustrated in FIG. 1.
FIG. 5 is an enlarged drawing of the membrane portion illustrated in FIG. 4.
FIG. 6 is a drawing illustrating the membrane portion before the framework portion is attached to the membrane portion.
FIG. 7 is an enlarged drawing of the membrane portion illustrated in FIG. 6.
FIG. 8 (A) and FIG. 8 (B) are drawings illustrating other examples of the shape of the framework portion.
FIG. 9 (A) is a drawing illustrating another example of the shape of the membrane portion and FIG. 9 (B) is a sectional drawing of the membrane portion illustrated in FIG. 9 (A).
FIG. 10 is a schematic drawing of a stent graft according to another embodiment of the present invention.
FIG. 11 (A) is an enlarged drawing of the stent graft illustrated in FIG. 10 and FIG. 11 (B) is a sectional drawing of the stent graft illustrated in FIG. 11 (A).
FIG. 12 is a schematic drawing of another stent graft as a comparative example.
FIG. 13 is a schematic drawing of another stent graft as a comparative example.

### DESCRIPTION OF THE EMBODIMENT

### <Embodiments>

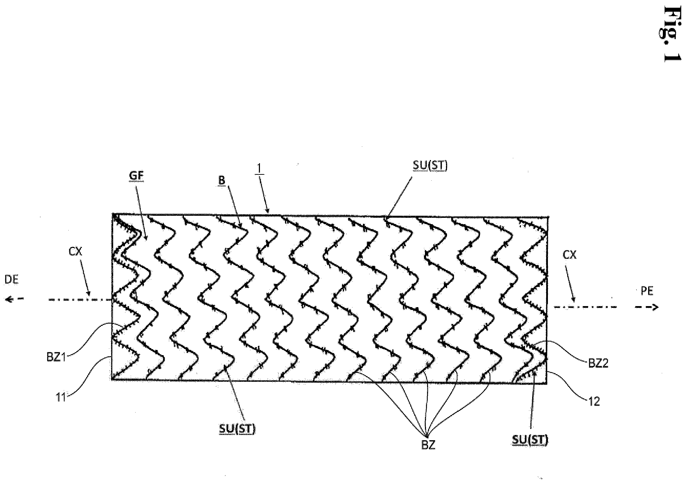

Hereinafter, a stent graft 1 according to an embodiment of the present invention will be explained with reference to the figures. In the following description, for convenience, the right side of the drawing in FIG. 1 or the like is referred to as a proximal end side (see an arrow PE in the figure), and the left side of the drawing is referred to as a distal end side (see an arrow DE in the figure). Note that, in a case that the stent graft 1 is placed in a living body lumen such as a blood vessel, the side closer to an operator such as a doctor corresponds to the proximal end side, and the side farther from the operator corresponds to the distal end side.

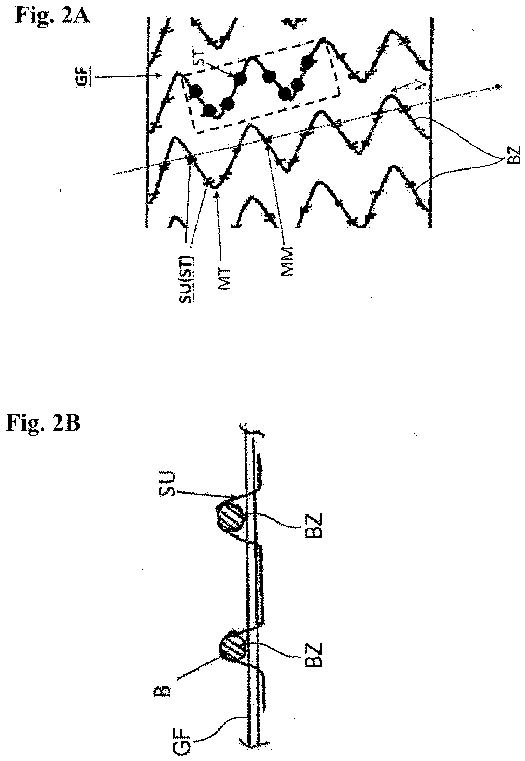

As illustrated FIG. 1, and FIG. 2 (A) to FIG. 2 (D), the stent graft 1 includes a framework portion B, a membrane portion GF, and a holding portion ST (specifically, suture SU described below) for holding the membrane portion GF onto the framework portion B.

The framework portion B is composed of a thin metal wire BZ (hereinafter, referred to as "strut BZ") having a shape spirally turning around a central axis CX of the stent graft 1 while folded in a zigzag pattern. More specifically, the strut BZ spirally turns with predetermined intervals in the central axis CX direction of the stent graft 1 while slightly inclining relative to the central axis CX direction of the stent graft 1. The framework portion B is expandable and contractable in a radial direction of the stent graft 1.

As a material constituting the strut BZ, a known metal or metal alloy, e.g. a Ti-Ni alloy, a stainless steel such as SUS316L, a shape memory alloy such as a Cu-Al-Mn alloy, a titanium alloy, and tantalum can be preferably used. The strut BZ can be manufactured by a method of laser-cutting an original plate made of these materials, a method of bending a wire rod made of these materials, or the like. Furthermore, a diamond-like carbon (DLC) and a fluorine-containing diamond-like carbon (FDLC), a polymer material such as urethane, a physiologically active substance such as heparin and urokinase, or an antithrombotic agent such as argatroban may be applied on the strut BZ as required.

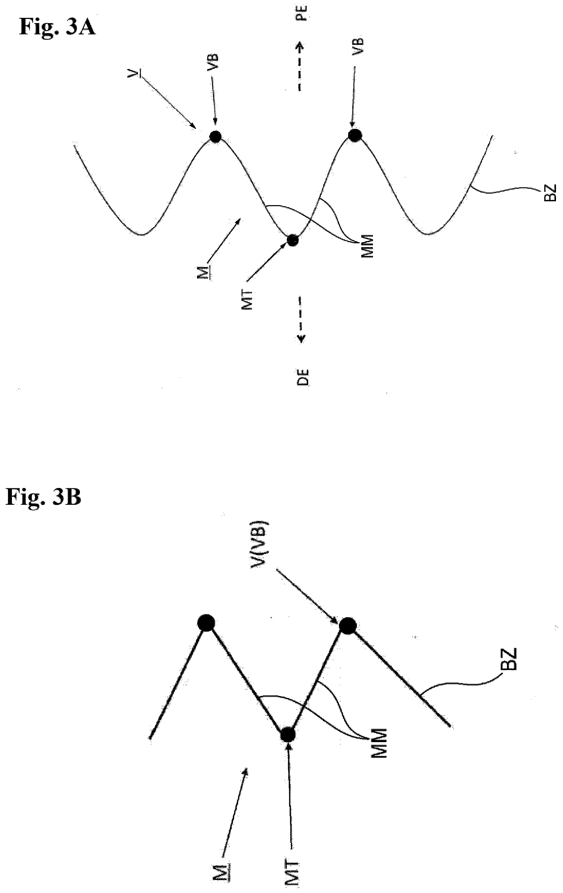

As illustrated in FIG. 3 (A), the strut BZ has a shape that mountain fold portions M bending so as to be convex toward the distal end side indicated by the arrow DE, and valley fold portions V bending so as to be convex toward the proximal end side indicated by the arrow PE are repeatedly formed in a circumferential direction. The shape illustrated in FIG. 3 (A) has inflection points between tops MT of the mountain fold portions M on the distal end side, and bottoms VB of the valley fold portions V on the proximal end side indicated by the arrow PE. That means, the strut BZ illustrated in FIG. 3 (A) has a shape that middle portions MM between the mountain fold portions M and the valley fold portions V are curved.

The shape of the strut BZ is not limited to the shape illustrated in FIG. 3 (A). For example, as illustrated in FIG. 3 (B), the strut BZ may be configured such that the tops MT of the mountain fold portions M and the bottoms VB of the valley fold portions V are connected not through the inflection points. That means, the strut BZ illustrated in FIG. 3 (B) is straight on the middle portions MM. Incidentally, in FIG. 3 (A) and FIG. 3 (B), the positions of the tops MT of the mountain fold portions M and the bottoms VB of the valley fold portions V are indicated by black circles.

As illustrated in FIG. 2 (A), on at least one position (two positions in this embodiment) on the middle portion MM, the strut BZ is sewn to and held on the membrane portion GF with the suture SU. However, as described later with reference to FIG. 2 (B) to FIG. 2 (D), the suture SU is fastened to the membrane portion GF such that the suture SU is in contact with at least a part of an outer peripheral face of the strut BZ. Note that, the suture SU does not completely block the strut BZ from moving. In other words, although the strut BZ is held on the membrane portion GF with the suture SU, the strut BZ can move relative to the membrane portion GF with a predetermined freedom in an axial direction of the strut BZ. In other words, the strut BZ is loosely attached to the membrane portion GF with the suture SU. Incidentally, such loose attachment can be achieved e.g. by arranging positions held with the suture SU on the middle portions MM of the strut BZ. In addition, the direction and the freedom degree of the movement of the strut BZ relative to the membrane portion GF can be arbitrarily adjusted depending on e.g. a number, positions, or the like of the sutured portions with the suture SU.

However, as illustrated in FIG. 1, a strut BZ1 held on a position closest to an end portion 11 of the distal end side of the stent graft 1 and a strut BZ2 held on a position closest to an end portion of the proximal end side 12 are held with more sutures SU than the sutures on the other struts BZ. That means, the struts BZ1 and BZ2 held on positions closest to both end portions 11 and 12 have more positions held with the suture SU than on the other struts BZ, and are held more firmly to the membrane portion GF. Thereby, wrinkles and the like are caused on the membrane portion GF at the distal end portion and the proximal end portion of the stent graft 1, so that blood can be prevented from entering between the stent graft 1 and a blood vessel wall.

In this embodiment, the strut BZ is held on the outer peripheral face of the membrane portion GF. However, the strut BZ may be held on the inner peripheral face of the membrane portion GF, or may be disposed inside the membrane portion GF by being sandwiched by the plurality of membrane portions GF. Furthermore, the struts BZ1 and BZ2 held on the both end portions 11 and 12 of the stent graft 1 are preferably held on the inner peripheral side of the membrane portion GF. Thereby, when the stent graft 1 is placed in a blood vessel, both end portions 11 and 12 of the stent graft 1 are separated from the blood vessel wall, blood can be prevented from entering between the stent graft 1 and the blood vessel wall.

As the method of holding the struts BZ1 and BZ2 on the inner peripheral side of the membrane portion GF, a method of sewing the struts BZ1 and BZ2 on the inner peripheral side of the membrane portion GF with the suture SU is used like the method of holding the strut BZ on the outer peripheral side of the membrane portion GF. As another method, a method in which the struts BZ1 and BZ2 are sewn on the outer peripheral side of the membrane portion GF and then the both end portions 11 and 12 of the stent graft 1 are folded inside can also be used. Furthermore, the struts BZ1 and BZ2 may be sandwiched between the membrane portions GF by sewing the struts BZ1 and BZ2 on the outer peripheral side of the membrane portion GF and then covering the struts BZ1 and BZ2 with a separately prepared membrane portion GF.

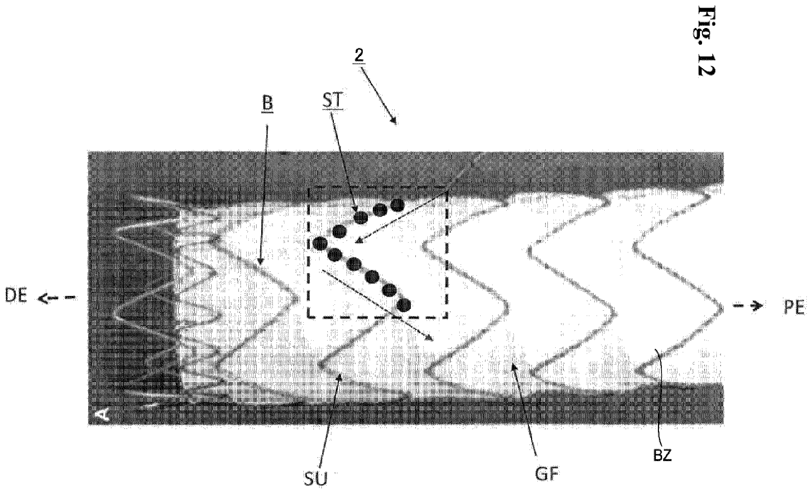

Although not illustrated in FIG. 1, a framework portion for delivering the stent graft 1 to a placement position in a blood vessel may be disposed on the end portion 11 of the distal end side of the stent graft 1 (also see FIG. 12 described below).

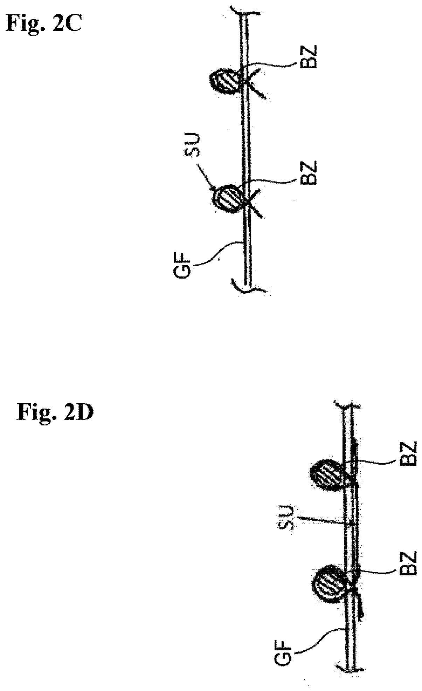

As illustrated in FIG. 2 (B), when holding the strut BZ on the membrane portion GF, the strut BZ may be sewn to the membrane portion GF such that the strut BZ is sandwiched between the suture SU and the membrane portion GF. Also, as illustrated in FIG. 2 (C) and FIG. 2 (D), the strut BZ may be sewn to the membrane portion GF such that the suture SU is wound around the outer peripheral face of the strut BZ. A plurality of sutures SU may be independent of each other for each held position as illustrated in FIG. 2 (C), or the suture SU may be continued over a plurality of held positions as illustrated in FIG. 2 (D).

The membrane portion GF is formed along the framework portion B composed of a plurality of struts BZ, and defines a hollow cylindrical portion in the inside of the framework portion. Preferably, the material constituting the membrane portion GF has a relatively high biocompatibility and durability and is chemically stable. Examples of the material suitable for the membrane portion GF include a fluororesin such as PTFE (polytetrafluoroethylene), silicone, a polyester resin such as polyurethane, polyethylene, polyester, and polyethylene terephthalate. Furthermore, biological materials such as silk thread, and blood vessels of other animals can also be used as the membrane portion GF. A film, a sheet, a fiber, a nonwoven fabric, or a woven fabric formed of these materials may be used to manufacture a single-layered or multi-layered membrane portion GF.

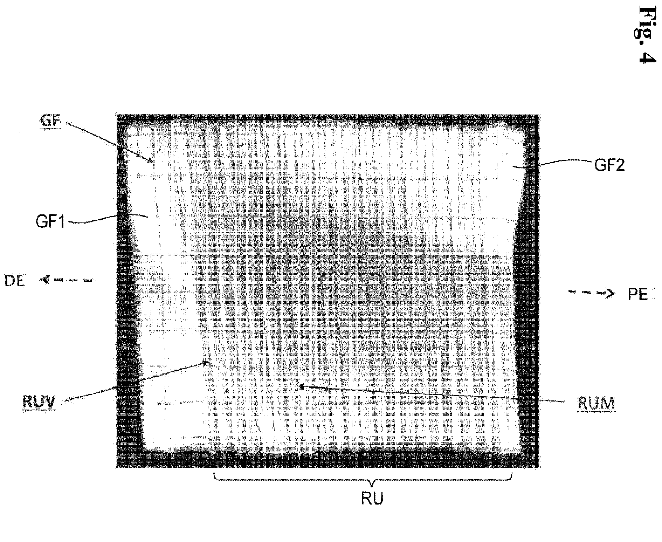

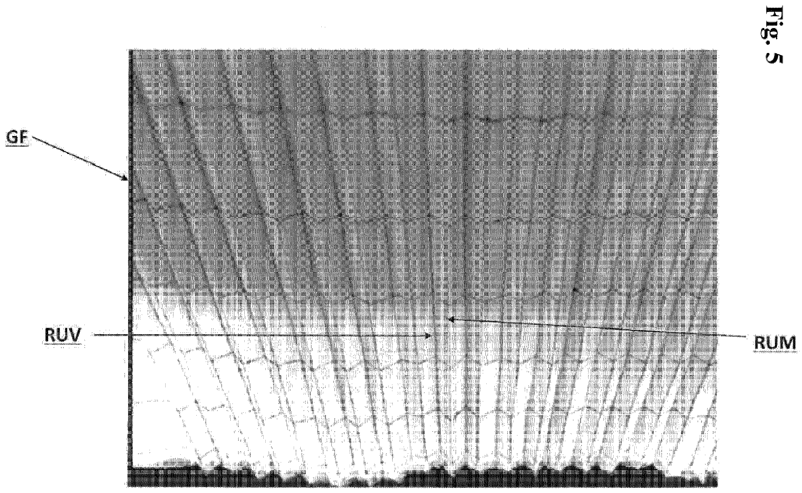

As illustrated in FIG. 4 and FIG. 5, the membrane portion GF has a textured structure in which mountain fold portions RUM and valley fold portions RUV extending in the circumferential direction of the stent graft 1 are regularly and repeatedly formed along the axial direction of the stent graft 1. In other words, the mountain fold portions RUM and the valley fold portions RUV are alternately repeated, so that a plurality of folds are formed on the membrane portion GF. These folds are formed on parts excluding the end portion of the distal end side GF1 and the end portion of the proximal end side GF2 in the membrane portion GF, but may also be formed on the whole membrane portion GF. Like the strut BZ, these folds extend so as to slightly incline relative to the axial direction of the stent graft 1. Such folds will be deformed more preferentially than the portion other than the folds of the membrane portion GF when the stent graft 1 bends such that the central axis CX of the stent graft 1 bends. From such an action, hereinafter, the folds of the membrane portion GF will be also referred to as "bent guide portions RU".

The shapes and the numbers of the mountain fold portions RUM and the valley fold portions RUV constituting the bent guide portions RU may be appropriately set depending on the flexibility required for the stent graft 1, the followability to blood vessels, and the like.

The membrane portion GF having the bent guide portions RU is formed e.g. through the following steps.
First, a film-like material for forming the membrane portion GF is cut out into a rectangular shape having a predetermined size. Then, one film-like material or a laminate of a plurality of film-like materials is heat-pressed using a mold corresponding to the shape of the bent guide portion RU. Thereby, the membrane portion GF having the bent guide portions RU is formed.

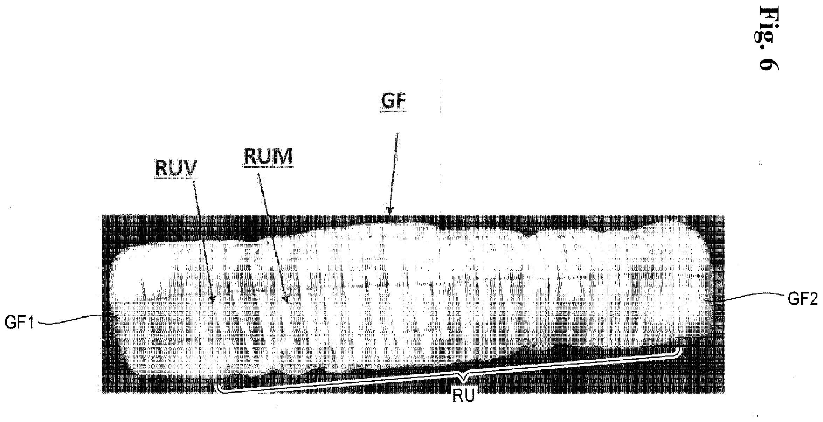

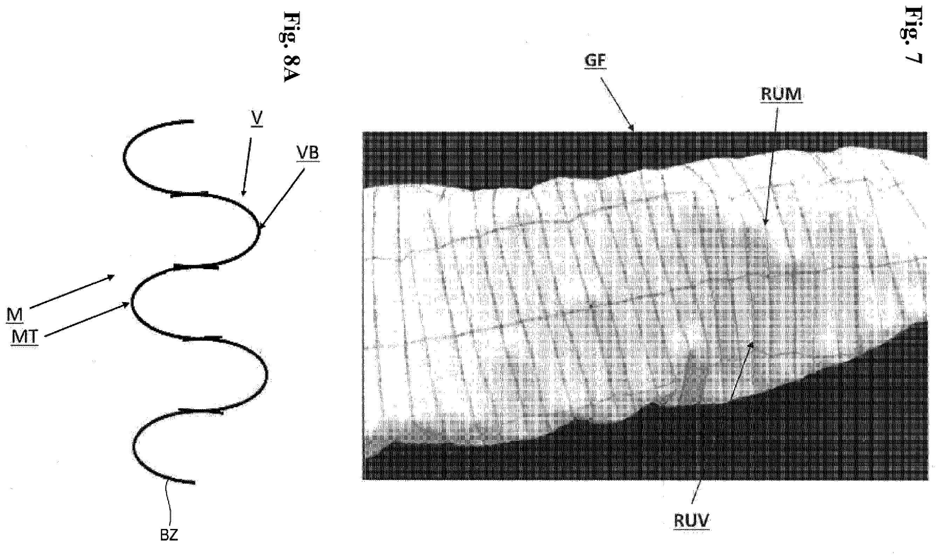

Furthermore, the membrane portion GF having the bent guide portions RU is rolled so as to move around the axis of the stent graft 1, and then both end portions in the circumferential direction are joined to each other by sewing, adhesion, or the like. Thereby, the cylindrical membrane portion GF is formed, as illustrated in FIG. 6 and FIG. 7. The membrane portion GF has the textured structure of the bent guide portions RU even when the membrane portion GF is formed in a cylindrical shape. The strut BZ is spirally wound around the outer peripheral face of the cylindrically formed membrane portion GF so as to be directed along the extending direction of the bent guide portions RU (i.e. folds), and is sewn to the membrane portion GF with the suture SU. In this case, as illustrated in FIG. 2 (A), in the vicinity of the top MT of the mountain fold portion M and on the middle portion MM, a seam of the suture SU is directed along the extending direction of the bent guide portion RU.

In the stent graft 1 according to this embodiment, the membrane portion GF has the aforementioned textured structure (i.e. the folds which are the bent guide portions RU), and thereby the membrane portion GF can be flexibly deformed when the stent graft 1 bends, and followability to blood vessels is improved.
As described above, the stent graft 1 can improve the followability to the living body lumen. Specifically, even when the stent is placed in a blood vessel or the like having a small radius of curvature at a curved position, collapse (so-called kink) of the framework portion B hardly occurs. Furthermore, unlike the conventional stent graft, each framework portion need not be individually manufactured for each living body lumen where the conventional stent graft is placed, and productivity of the stent graft 1 can be increased. That means, both the followability to the living body lumen and the productivity in manufacturing can be achieved.

Furthermore, although the strut BZ of the framework portion B is held on the membrane portion GF with the suture SU, the strut BZ is movable relative to the membrane portion GF in the axial direction of the strut BZ with a predetermined degree of freedom. This makes it possible not only to deform the whole stent graft 1 in the axial direction, but also to change the presence and the degree of the relative movement between the strut BZ and the membrane portion GF at each sutured position of the strut BZ. The whole stent graft 1 can be flexibly deformed in any direction compared to the case that the strut BZ and the membrane portion GF are difficult to move relative to each other.

### <Comparative Examples>

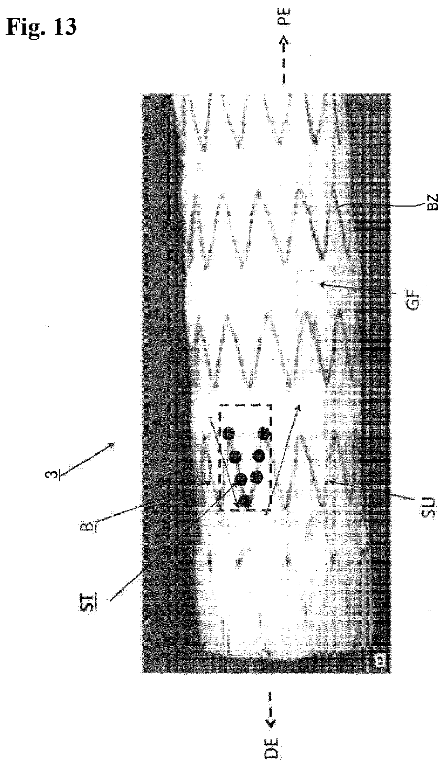

Next, the stent grafts 2 and 3 as comparative examples will be briefly explained.
As illustrated in FIG. 12 and FIG. 13, in the stent grafts 2 and 3, the strut BZ constituting the framework portion B is sewn to the membrane portion GF with the suture SU (see the holding portion ST in the figure). However, compared to the aforementioned embodiment, these comparative examples have more positions held with the suture SU, and the whole strut BZ is firmly held on the membrane portion GF. Thus, in the stent grafts 2 and 3, the relative movement between the membrane portion GF and the strut BZ is not substantially caused. As a result, the stent grafts 2 and 3 are inferior in the followability to the blood vessels compared to the stent grafts 1 and 1A according to the aforementioned embodiment.

### <Other Aspects>

Note that the present invention is not limited to each of the aforementioned embodiments, and various modifications can be adopted within the scope of the present invention. For example, the present invention is not limited to the aforementioned embodiments, and optionally deformation, improvement, or the like can be added. Additionally, the material, shape, dimension, number, arrangement position, and the like of each constituent in the aforementioned embodiments are arbitrarily decided and not limited as long as the present invention can be achieved.

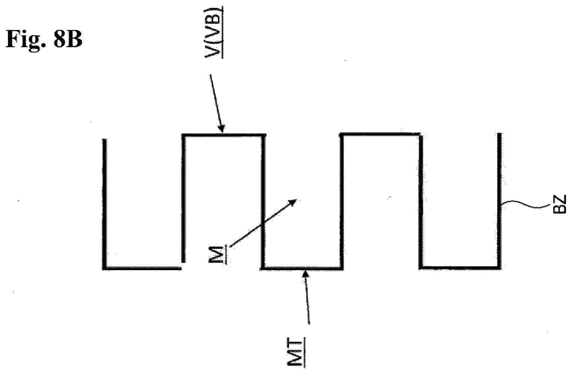

For example, in the aforementioned embodiments, the strut BZ has an annular shape extending in the circumferential direction of the stent graph 1 while folded in a zigzag pattern. However, the strut BZ may have a shape that a curved or bent unit shape as illustrated in FIG. 8 (A) and FIG. 8 (B) is repeated in the circumferential direction. In this case, members corresponding to the individual unit shapes can be joined by means of caulking pipe, welding or the like. In addition, the framework portion B may be composed of a plurality of unit annular bodies that a thin metal wire is formed into an annular shape circumferentially wound around the stent graft 1 while folded in a zigzag pattern. In this case, the individual struts BZ are independent of each other, unlike the spiral strut BZ in the aforementioned embodiments.

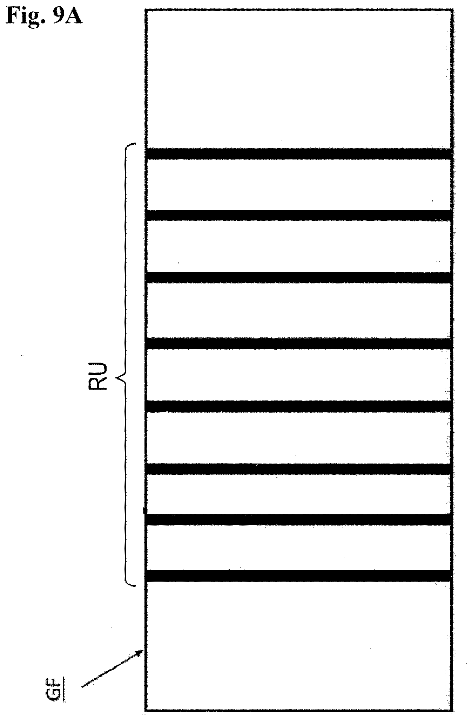

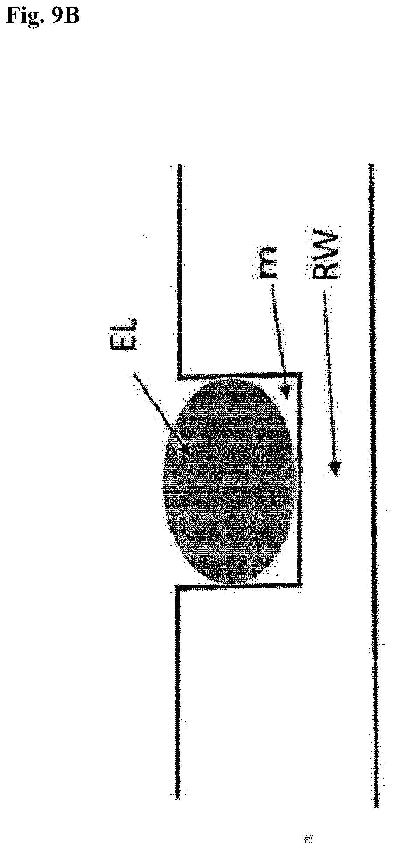

Furthermore, as the bent guide portions RU, a plurality of grooves m (in other words, thin portions RW) extending in the circumferential direction of the stent graft 1 may be repeatedly provided in the axial direction of the stent graft 1, as illustrated in FIG. 9 (A) and FIG. 9 (B). In addition, elastic bodies EL (e.g. silicone resin or the like) may be embedded in these grooves m (thin portions RW).

Furthermore, the bent guide portions RU are not necessarily formed so as to extend in the circumferential direction of the stent graft 1. For example, the bent guide portions RU may be configured such that a plurality of unit guide portions having a rhomboidal shape or the like are regularly formed on the membrane portion GF. In this case, the peripheral edge of the unit guide portion is preferably processed such that the membrane portion GF can be easily bent (e.g. the thickness of the membrane portion GF is decreased on the peripheral edge of the unit guide portion).

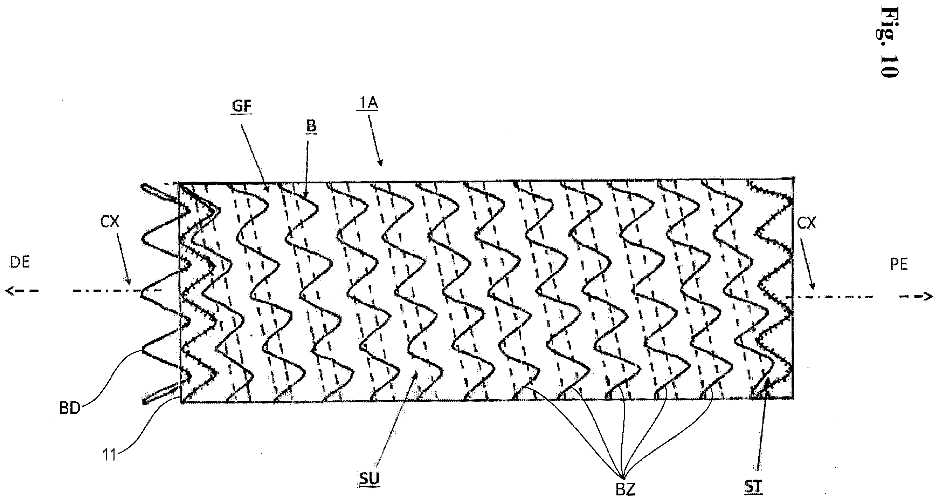

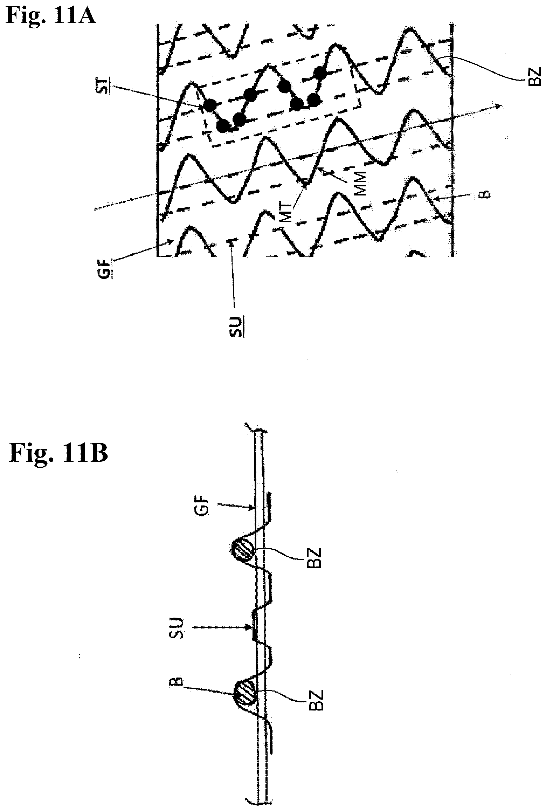

Furthermore, as illustrated in FIG. 10 and FIG. 11, the suture SU may be sewn to the membrane portion GF with predetermined intervals along the circumferential direction of the stent graft 1A regardless of the arrangement of the strut BZ. Even if the arrangement of the strut BZ is not actively taken into account, the strut BZ can enter the stitches of the suture SU at a pitch allowing the strut BZ to be held on the membrane portion GF, by properly defining intervals for disposing the suture SU, or the like. Incidentally, the stent graft 1A include stitches in which the strut BZ intervenes between the suture SU and the membrane portion GF, and stitches in which the strut BZ does not intervene between the suture SU and the membrane portion GF. Compared to the stent graft 1 according to the aforementioned embodiments, the stent graft 1A facilitates the step of holding the strut BZ with the suture SU and improves the productivity of the stent graft.

Additionally, in the aforementioned embodiments, the strut BZ is spirally wound around the outer peripheral face of the membrane portion GF so as to be directed along the extending direction of the bent guide portions RU, and is sewn with the suture SU, so that the seam of the suture SU is directed along the extending direction of the bent guide portions RU. However, this case is merely an example and the present invention is not limited to this case. For example, the extending direction of the bent guide portions RU and the direction of the seam of the suture SU may be different from each other.

Furthermore, as illustrated in FIG. 10, a framework portion BD for delivery may be disposed on the end portion 11 of the distal end side of the stent graft 1A. Like the strut BZ, the framework portion BD may be held on the membrane portion GF with the suture SU.

Furthermore, the strut BZ may be fixed to the membrane portion GF with a ribbon-like tape or a metal wire rod instead of the suture SU.

This application is based on Japanese Patent Application filed on October 16, 2017 (Japanese Patent Application No. 2017-200237), of which the contents are incorporated in this specification by reference.

### INDUSTRIAL APPLICABILITY

The stent graft according to the present invention makes it possible to achieve both followability to a blood vessel and productivity in manufacture. The present invention having this effect can be used for treating e.g. aortic aneurysm.

### [Reference Signs List]

- 1, 1A...: Stent graft
- B...: Stent (framework portion)
- BZ...: Strut
- GF...: Graft (membrane portion)
- RU...: Bent guide portion
- SU...: Suture

## Claims

1. A stent graft having a ductal shape, the stent graft comprising:
a framework portion expandable and contractable along a radial direction of the stent graft; and
a membrane portion provided along the framework portion, wherein
the membrane portion includes a bent guide portion which has a textured structure formed along the radial direction being regularly arranged and is deformable more preferentially than other positions in the membrane portion when the stent graft bends.

2. The stent graft according to claim 1, wherein the textured structure extends along a circumferential direction of the stent graft and is regularly arranged along an axial direction of the stent graft.

3. The stent graft according to claim 2, wherein the textured structure is formed by folds provided on the membrane portion.

4. The stent graft according to claim 2, wherein the textured structure is formed by at least one of grooves provided on the membrane portion, and elastic bodies disposed in the grooves.

5. The stent graft according to any one of claims 1 to 4, wherein the bent guide portion is arranged so as to extend in a direction corresponding to an arrangement of the framework portion.

6. The stent graft according to any one of claims 1 to 5, wherein the framework portion has a spiral shape turning around an axis of the stent graft while being folded in a zigzag pattern, or a spiral shape turning around the axis of the stent graft while a curved or bent unit shape is repeated.

7. The stent graft according to any one of claims 1 to 6, wherein the framework portion has a linear shape and is held on the membrane portion in a state where the framework portion is movable relative to the membrane portion at least in an axial direction of the framework portion when the stent graft bends.
